# EUROPÄISCHE PATENTANMELDUNG

(11) **EP 4 268 767 A1**
(43) Veröffentlichungstag der Anmeldung: **01.11.2023**
(21) Anmeldenummer: 22170330.9
(22) Anmeldetag: 27.04.2022
(51) Int. Cl.: A61C 13/08, A61C 19/10, A61C 13/083, A61C 13/09

(54) **VERFAHREN ZUR HERSTELLUNG EINER DENTALEN RESTAURATION MIT EINEM ZIELFARBWERT**

(71) Anmelder: VITA-ZAHNFABRIK H. Rauter GmbH & Co. KG, 79713 Bad Säckingen (DE)
(72) Erfinder: Gödiker, Michael, 79713 Bad Säckingen (DE); Gödiker, Berit, 79713 Bad Säckingen (DE)
(74) Vertreter: dompatent von Kreisler Selting Werner - Partnerschaft von Patent- und Rechtsanwälten mbB

(57) **Zusammenfassung**

Die vorliegende Erfindung betrifft ein Verfahren zur Herstellung einer dentalen Restauration mit einem Zielfarbwert, wobei der Zielfarbwert durch Glasieren eines Formkörpers und Wärmebehandeln des glasierten Formkörpers erreicht wird.

## Beschreibung

Die vorliegende Erfindung betrifft ein Verfahren zur Herstellung einer dentalen Restauration mit einem Zielfarbwert, wobei der Zielfarbwert durch Glasieren eines Formkörpers und Wärmebehandeln des glasierten Formkörpers erreicht wird.

Der Begriff der restaurativen Zahnmedizin bezeichnet die Behandlungen von Zahnproblemen bei gleichzeitig funktioneller und ästhetischer Wiederherstellung der Zähne. Dabei steht insbesondere das naturgetreue Nachempfinden der Zähne im Vordergrund, das in vielen Fällen über die ästhetische Akzeptanz einer dentalen Restauration entscheidet. Zu diesem Zweck stehen in der Regel eine Reihe von Grundfarben zur Verfügung, die farblich an die bestehende Zahnsituation angepasst werden.

Die konventionelle Farbbestimmung von Zähnen erfolgt in der Regel mit Hilfe sogenannter Farbringe oder -skalen, die mit Farbmustern versehen sind und zum visuellen Farbabgleich dienen, wobei die Zusammenstellung der Farbmuster traditionell den am häufigsten in der Natur vorkommenden Zahnfarben, welche empirisch ermittelt wurden, entspricht.

In diesem Zusammenhang beschreibt DE 693 19 291 eine Farbmischungs-Indikatorvorrichtung, die bei der Auswahl von gewünschten künstlichen Zahnfarben bei der Herstellung von Brücken, Kronen, Teilgebissen und dergleichen Anwendung findet. Offenbart wird ein Set von Zahnfarbenmischungs-Indikatorvorrichtungen, wobei jede beteiligte Indikatorvorrichtung eine Mehrzahl von Farbmustern aufweist, die in einer Anordnung vorgesehen sind und um ein zentrales Muster der Farbmuster angeordnet sind, wobei jedes Muster von diesen benachbarten Mustern beabstandet ist, wobei die Farben der Muster Farben eines vorbestimmten Farbsystems darstellen, das ein dreidimensionales Koordinatensystem (L, a, b) aufweist, das mit der genannten Anordnung ausgewichtet ist, wobei die Farbe jedes Musters der Farbe an der entsprechenden Position in dem Koordinatensystem (L, a, b) entspricht, wobei die Farben der Muster jeder beteiligten Vorrichtung in einer Ebene des Koordinatensystems liegen und die jeder Vorrichtung entsprechende Ebene im Wesentlichen parallel zu und im Abstand von den Ebenen der anderen beteiligte Vorrichtungen angeordnet ist, und wobei im Gebrauch des Sets ein Zahn eines Patienten mit jeglicher der beteiligten Vorrichtungen verglichen werden kann und eine gewünschte Zahnfarbe zwischen einem ausgewählten Paar von Farbmustern zur Herstellung gemischt werden kann, indem eine Mischung der Farbmaterialien gebildet wird, die zur Herstellung des ausgewählten Paares verwendet werden, wobei die zentralen Farbmuster einander benachbarter Ebenen entlang einer gemeinsamen Linie angeordnet sind und in Bezug auf eine Achse (L) des Koordinatensystems versetzt sind, die zu einander benachbarter Ebenen senkrecht sind.

Mit Hilfe solcher Farbskalen erfolgt eine schrittweise Bestimmung der Zahngrundfarbe gemäß den drei Farbdimensionen Helligkeit, Farbintensität und Farbton, die den menschlichen Farbeindruck bestimmen. Entsprechende Farbskalen sind beispielsweise unter der Bezeichnung VITA SYSTEM 3D-MASTER^{®} kommerziell erhältlich.

Um eine objektivere Erfassung der Zahnfarbe zu erzielen, schlägt der Stand der Technik eine Reihe von technisch unterstützten Verfahren vor.

EP 3 643 272 beschreibt ein Verfahren zur Festlegung einer Zahnfarbe einer Füllung oder sonstigen Restauration, die insbesondere unter Verwendung von Composite-Materialien hergestellt wird, wobei mindestens ein Zahn hinsichtlich seines Farbwerts elektronisch erfasst, insbesondere gescannt wird, wobei natürliche Zähne vorab spektral gemessen werden, wobei die gemessenen Farbwerte in einem Farbraum in mehrere, insbesondere vier, Kategorien, die in dem Farbraum je eine dreidimensionale Farbwolke bilden, eingeteilt werden, und zwar mittels eines strukturerkennenden Algorithmus, und wobei die Zahnfarbe der Füllung festgelegt wird, indem ermittelt wird, zu welcher der Kategorien der Zahn den geringsten Farbabstand hat oder in welche Farbwolke der Zahn fällt.

WO 2020/156855 offenbart ein Unterstützungssystem zur Zahnbehandlung, insbesondere durch Veränderung der Zahnfarbe, mit einem Messgerät, welches die Zahndaten zu behandelnder Zähne, insbesondere die Zahnfarbe sowie Zahnkoordinaten, bestimmt, einer Datenverarbeitungseinrichtung sowie einer Kamera zur Aufnahme eines Zahnbilds, wobei die Datenverarbeitungseinrichtung vorzugsweise ein Empfangsmodul und das Messgerät ein Sendemodul zur Übertragung gemessener Zahndaten vom Messgerät an die Datenverarbeitungseinrichtung aufweist, einem in die Datenverarbeitungseinrichtung integrierten Zuordnungsmodul zur Zuordnung der empfangenen Zahndaten zu einem Zahnbild, einer Auswahleinrichtung zum Auswählen eines Farbveränderungsgrades und einer Anpassungsvorrichtung zur Anpassung des Zahnbilds an den ausgewählten Farbveränderungsgrad.

EP 3 650 821 betrifft ein System zur Bestimmung der Farbe von Zähnen, das eine 2D- oder 3D-Kamera umfasst, wobei das System angepasst ist, um Bilder zu erfassen, die durch die Kamera aufgenommen werden, und einen Aufsatz, wobei der Aufsatz einen Abstandshalter umfasst, der eine Verbindungeinrichtung zum Anbringen des Aufsatzes an der Kamera aufweist, wobei der Abstandshalter eine Blende umfasst, mittels der die Kamera angepasst ist, um ein Bild von Zähnen aufzunehmen, wobei der Abstandshalter angepasst ist, um einen Abstand zwischen einer Aufnahmevorrichtung der Kamera und der Zahnoberfläche zu erzeugen, und wobei der Abstandshalter angepasst ist, um einen Abstand zwischen der Aufnahmevorrichtung der Kamera und der Blende zu erzeugen, wobei die Kamera einen normalen Aufzeichnungsmodus ohne die Verwendung des Aufsatzes und einen Farbmessmodus zur Verwendung mit einem Aufsatz zum Messen der Farbe des Zahns aufweist, wobei das System zum Erkennen des Vorhandenseins des Aufsatzes auf der Kamera auf der Basis der Änderungen in dem durch die Kamera aufgenommenen Bildfeld angepasst ist, um in einen Farbmessmodus umzuschalten, wenn diese Änderungen im Bildfeld detektiert werden.

WO 2020/154450 bezeichnet ein Verfahren zur Farbanpassung, das die folgenden Schritte umfasst: Bereitstellen von Zahnmessdaten, wobei die Zahnmessdaten mindestens einen Einsatzbereich umfassen, in den die Zahnrestauration eingesetzt werden soll; Bereitstellen von Restaurationsdaten; Bereitstellen von Informationen über Färbe- und Glasurfarben; Erzeugen einer Farbcharakteristik aus den Zahnmessdaten oder anderen Informationen unter Berücksichtigung der Restaurationsdaten und Berechnen einer mehrfarbigen Zielfärbung aus der Farbcharakteristik unter Berücksichtigung von Informationen über Färbe- oder Glasurfarben, und Ausgeben der mehrfarbigen Zielfärbung zur Farbanpassung der Zahnrestauration.

Sowohl die konventionelle, also die subjektiv-visuelle Zahnfarbenstimmung, als auch die technisch unterstützte objektivierte Zahnfarbmessung werden durch viele Einflussfaktoren bestimmt, so dass das ästhetische Ergebnis oft von dem Können und der Erfahrung des Zahnarztes und des Zahntechnikers abhängt. Weiterhin weist das bestehende System den Nachteil auf, dass eine große Anzahl von Proben vorgehalten werden muss, um eine möglichst farbgetreue Anpassung zu erzielen.

In den Fällen, in denen eine falsche Wahl getroffen wurde, müssen außerdem aufwendige Farbanpassungen und -korrekturen vorgenommen werden.

Insofern besteht weiterhin der Bedarf nach einem vereinfachten und verlässlichen System zur Auswahl und Herstellung dentaler Restaurationen mit einer natürlichen Ästhetik, die sich in das bestehende Zahnschema einpassen. Darüber hinaus ist es wünschenswert, eine einfache Anpassung der Zahnfarbe vornehmen zu können.

Vor diesem Hintergrund stellt die vorliegende Erfindung ein Verfahren zur Herstellung einer dentalen Restauration bereit, das eine einfache und effiziente Farbanpassung erlaubt und durch das die Anzahl an Proben, die für eine genaue Farbanpassung vorgehalten werden muss, deutlich reduziert werden kann.

Ein erster Gegenstand der vorliegenden Erfindung ist daher ein Verfahren zur Herstellung einer dentalen Restauration mit einem Zielfarbwert FW(Z), das die folgenden Schritte umfasst:
a) Bereitstellen eines Formkörpers für dentale Restaurationen, wobei der Formkörper einen Grundfarbwert FW(F) aufweist und wobei der Grundfarbwert FW(F) des Formkörpers bezüglich des Zielfarbwerts FW(Z) der dentalen Restauration einen maximalen Differenzwert ΔE von 5 aufweist;
b) Aufbringen einer Glasur auf den Formkörper, wobei die Glasur einen Farbwert FW(G) aufweist; und
c) Wärmebehandeln des glasierten Formkörpers unter Erhalt der dentalen Restauration,
dadurch gekennzeichnet, dass der Farbwert FW(G) der Glasur der Differenz ΔE zwischen den Farbwerten FW(Z) und FW(F) entspricht.

Bei den Farbwerten und Differenzwerten handelt es sich vorzugsweise um CIEL^{∗}a^{∗}b^{∗}-Werte, wobei sich die Differenz vorzugsweise auf jeweils einen der Werte bezieht. Die Bestimmung solcher Werte ist dem Fachmann bekannt und beispielsweise von A. Baltzer und V. Kaufmann-Jinoian in "Die Bestimmung der Zahnfarben", erschienen in QZ Quintessenz Zahntechnik, 30. Jahrgang, Juli 2004, beschrieben.

In den Fällen, in denen keine passende Zahnfarbe zur Verfügung steht oder vom Anwender eine falsche Auswahl getroffen wurde, sind oftmals manuelle Farbkorrekturen notwendig. Dazu muss zunächst die richtige keramische Malfarbe mit Pinsel oder Spray an der richtigen Stelle aufgebracht werden, was eine gewisse Erfahrung voraussetzt. Darauf erfolgt ein sogenannter Malfarbenfixierbrand. Anschließend wird eine Glasur aufgetragen und ein Glasurbrand durchgeführt, erst dann kann die Farbwirkung definitiv beurteilt werden. Durch das erfindungsgemäße Verfahren kann die farbliche Anpassung mit nur einem Brand erfolgen, wodurch sich das Ergebnis schneller beurteilen lässt.

Im Rahmen des erfindungsgemäßen Verfahrens erfolgt die Bereitstellung des Formkörpers vorzugsweise durch Auswahl aus einem Set von Formkörpern mit jeweils einem definierten Grundfarbwert FW(F) durch Vergleich mit den zur Restauration benachbarten Zähnen im Mund des Patienten, vorzugsweise mit Hilfe eines Farbrings zur Zahnfarbenbestimmung. Durch diese Art der Bereitstellung in Kombination mit der farblichen Anpassung durch Auftragen einer ausgewählten Glasur ist es möglich, ein begrenztes Angebot an unterschiedlichen Grundfarben bereitzuhalten, deren Farbe durch eine entsprechende Glasur angepasst werden kann. Die Notwendigkeit ein komplettes Farbsortiment an Rohlingen vorzuhalten entfällt somit. Vielmehr besteht nun die Möglichkeit, mit Hilfe unterschiedlicher farblicher Glasuren ausgehend von definierten Ausgangspunkten bestimmte Farborte zu erreichen.

Um den Einfluss der subjektiven Wahrnehmung von Farben bei der Farbauswahl zu minimieren, hat es sich als vorteilhaft erwiesen, digitale Unterstützung heranzuziehen. Daher ist eine Ausführungsform des erfindungsgemäßen Verfahrens bevorzugt, in der die Bestimmung des ΔE und/oder die Auswahl des Formkörpers computergestützt erfolgt.

Gemäß dem erfindungsgemäßen Verfahren wird der Zielfarbwert FW(Z) der dentalen Restauration durch Auftrag einer entsprechenden Glasur erreicht. Hierbei hat es sich als vorteilhaft erwiesen, die Dicke, in der die Glasur aufgetragen wird zu begrenzen, um so eine Beeinträchtigung des Farbergebnisses zu minimieren. In einer bevorzugten Ausführungsform des erfindungsgemäßen Verfahrens wird die Glasur daher in einer Dicke von 10 bis 50 µm, vorzugsweise 20 bis 40 µm auf den Formkörper aufgetragen. Das Auftragen der Glasur kann dabei mit dem Fachmann bekannten Verfahren manuell oder maschinell erfolgen.

Das erfindungsgemäße Verfahren verwendet Glasuren mit bestimmten Farbwerten, um eine Anpassung der Farbe eines bereitgestellten Formkörpers zu erreichen. Ein weiterer Gegenstand der vorliegenden Erfindung ist daher eine Glasur zur Verwendung in dem erfindungsgemäßen Verfahren, wobei die Glasur
i) eine flüssige Anmischphase; und
ii) eine feste Phase umfassend färbende Substanzen
aufweist, wobei der Anteil der flüssigen Anmischphase in der Glasur mindestens 60 Gew.-% beträgt, bezogen auf das Gesamtgewicht der Glasur und wobei die Glasur einen definierten Farbwert FW(G) aufweist.

Der definierte Farbwert FW(G) der Glasur entspricht vorzugsweise der Differenz ΔE zwischen einem Grundfarbwert FW(F) eines Formkörpers und dem Zielfarbwert FW(Z) einer aus diesem Formkörper herzustellenden dentalen Restauration.

Die Anmischphase ist vorzugsweise ausgewählt aus der Gruppe bestehend aus Wasser, einem oder mehreren Alkoholen und Mischungen hiervon. Der Alkohol ist vorzugsweise ausgewählt aus der Gruppe der 1-, 2- und 3-wertigen Alkohole sowie Mischungen hiervon. In einer besonders bevorzugten Ausführungsform ist der Alkohol ausgewählt aus der Gruppe bestehend aus Glycerin, 1,3-Butandiol, 1,2-Propandiol, Ethanol und Mischungen hiervon.

In einer bevorzugten Ausführungsform umfasst die Anmischphase weiterhin einen oder mehrere Komplexbildner, vorzugsweise ausgewählt aus der Gruppe bestehend aus Ethylendiamintetraessigsäure und Citronensäure.

Als Anmischphase können solche verwendet, die kommerziell erhältlich sind, wie beispielsweise Triplex^{®} Lösung B.

Als feste Phase der Glasur können im Stand der Technik bekannte Zusammensetzungen verwendet werden. Vorzugsweise wird bei der Glasur als feste Phase auf eine Grundmischung zurückgegriffen, der je nach Bedarf färbende Substanzen beigemischt werden. In einer bevorzugten Ausführungsform umfasst die feste Phase der Glasur 45 bis 70 Gew.-% SiO₂, besonders bevorzugt 50 bis 65 Gew.-%, bezogen auf das Gesamtgewicht der festen Phase. Weiterhin bevorzugt ist eine Ausführungsform, in der die feste Phase eine oder mehrere der folgenden Komponenten umfasst, jeweils bezogen auf das Gesamtgewicht der festen Phase:
- 5 bis 15 Gew.-%, vorzugsweise 7 bis 13 Gew.-% Al₂O₃;
- 5 bis 15 Gew.-%, vorzugsweise 7 bis 13 Gew.-% B₂O₃;
- 5 bis 15 Gew.-%, vorzugsweise 7 bis 13 Gew.-% K₂O;
- 5 bis 15 Gew.-%, vorzugsweise 7 bis 13 Gew.-% Na₂O.

Um die gewünschten Farbwerte zu erreichen kann die feste Phase bis zu 5 Gew.-% an färbenden Substanzen umfassen, bezogen auf das Gesamtgewicht der festen Phase.

Als färbende Substanzen können in der Branche übliche Pigmente und Pigmentmischungen eingesetzt werden, wie sie in der Keramik-, Glas- und Porzellanindustrie verwendet werden. Insofern weist die erfindungsgemäße Glasur eine hohe Kompatibilität mit bestehenden Systemen auf.

In einer weiterhin bevorzugten Ausführungsform liegt die Glasur vorzugsweise als Kit vor, umfassend einen Behälter mit der Anmischphase und einen Behälter mit der festen Phase umfassend färbende Substanzen. Diese Form der Bereitstellung bietet den Vorteil, dass die gewünschte Glasur vor Ort angerührt werden kann und einem Eintrocknen durch Lagerung vorgebeugt wird.

Die vorliegende Erfindung hat das Ziel, die Herstellung von dentalen Restaurationen mit gewünschten Farbwerten zu erleichtern. In diesem Zusammenhang ist ein weiterer Gegenstand der vorliegenden Erfindung ein Kit zur Herstellung einer dentalen Restauration umfassend
a) ein Sortiment an Glasuren für Formkörper für dentale Restaurationen, wobei jede Glasur einen definierten Farbwert FW(Gₓ) aufweist, wobei die Farbwerte FW (Gₓ) den Differenzen ΔE zwischen den Farbmustern eines standardisierten Farbrings zur Zahnfarbenbestimmung entsprechen und wobei ΔE nicht größer als 5 ist, und
b) einen Farbführer zur Zuordnung eines ermittelten ΔE-Werts zu der entsprechenden Glasur.

Das erfindungsgemäße Kit findet vorzugsweise in dem erfindungsgemäßen Verfahren Anwendung und erlaubt es, ausgehend von einem Formkörper mit einem Grundfarbwert FW(F) durch Auswahl einer geeigneten Glasur auf eine einfache Weise und mit nur einer Wärmebehandlung eine dentale Restauration mit einem Zielfarbwert FW(Z) zu erreichen, wobei der Farbwert FW(G) der Glasur der Differenz ΔE zwischen dem Grundfarbwert FW(F) des Formkörpers und dem Zielfarbwert FW(Z) der dentalen Restauration entspricht. Das erfindungsgemäße Kit ermöglicht durch die Bereitstellung eines Farbführers eine gezielte Auswahl der geeigneten Glasur, die der ermittelten Farbwertdifferenz ΔE zwischen Grundfarbwert FW(F) und Zielfarbwert FW(Z) entspricht. Durch Auftragen der Glasur und Wärmebehandeln kann somit der Farbunterschied ausgeglichen werden und eine dentale Restauration zur Verfügung gestellt werden, die sich ästhetisch in das bestehende Zahnschema des Patienten einfügt.

Das erfindungsgemäße Kit ermöglicht es, durch eine begrenzte Anzahl von Glasuren ein breites Farbspektrum zu erreichen. In einer bevorzugten Ausführungsform umfasst das Kit ein Sortiment von 2 oder mehr, vorzugsweise 3 oder mehr, besonders bevorzugt 4 oder mehr Glasuren und/oder nicht mehr als 10, vorzugsweise nicht mehr als 8, besonders bevorzugt nicht mehr als 6 Glasuren.

In einer weiterhin bevorzugten Ausführungsform umfasst das Kit weiterhin einen Applikator zum Aufbringen der Glasur auf einen Formkörper für dentale Restaurationen. In einer ebenfalls bevorzugten Ausführungsform umfasst das erfindungsgemäße Kit neben dem Sortiment an Glasuren, dem Farbführer und optional dem Applikator weiterhin ein Sortiment an Formkörpern für dentale Restaurationen mit jeweils einem Grundfarbwert FW(Fₓ), die vorzugsweise den Farbwerten eines standardisierten Farbrings zur Zahnfarbenbestimmung entsprechen. Auf diese Weise kann ein Komplettpaket zur Verfügung gestellt werden, das eine einfache und effiziente Herstellung dentaler Restaurationen erlaubt.

Vorzugsweise handelt es sich bei den Glasuren des erfindungsgemäßen Kits um solche gemäß der vorliegenden Erfindung.

Ein weiterer Gegenstand der vorliegenden Erfindung ist die Verwendung einer Glasur mit einem definierten Farbwert FW(G) in der Herstellung dentaler Restaurationen, wobei der Farbwert FW(G) der Differenz ΔE zwischen den Farbmustern eines standardisierten Farbrings zur Zahnfarbenbestimmung entspricht, wobei ΔE nicht größer als 5 ist.

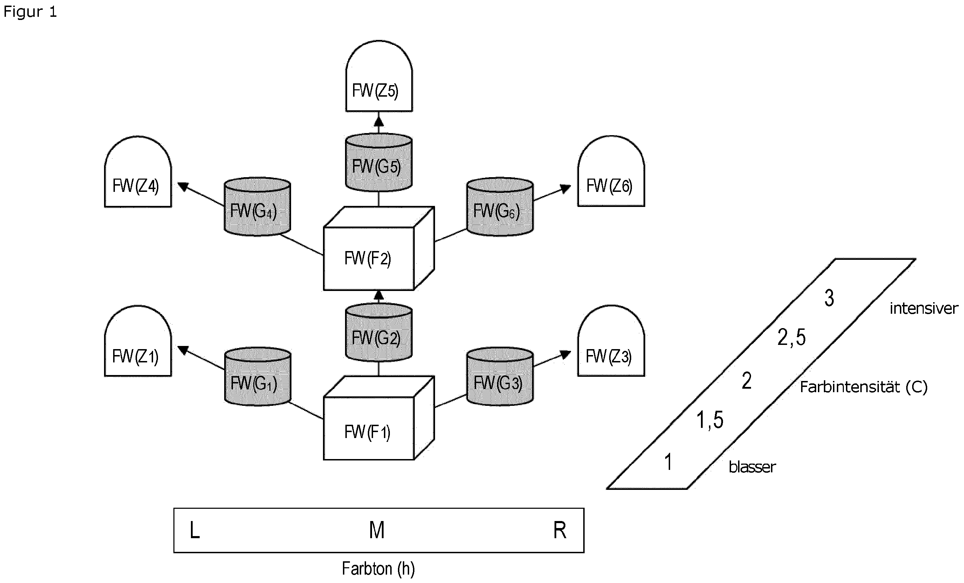

Figur 1 verdeutlicht die vorliegende Erfindung, ist jedoch keineswegs als Einschränkung des Erfindungsgedanken zu verstehen.

Ausgehend von einer keramischen Restauration mit einem Grundfarbwert, beispielsweise FW(F₁), ermöglicht die vorliegende Erfindung eine einfache und effiziente Farbanpassung auf einen Zielfarbwert FW(Z₁) durch Aufbringen einer Glasur, deren Farbwert FW(G₁) der Differenz der Farbwerte FW(F₁) und FW(Z₁) entspricht. Je nach Anforderung kann dies einstufig oder mehrstufig erfolgen. Die Auswahl geeigneter Glasuren erfolgt anhand einer anerkannten Farbwerteskala, unter Berücksichtigung des Farbtons und der Farbintensität.

## Patentansprüche

1. Verfahren zur Herstellung einer dentalen Restauration mit einem Zielfarbwert FW(Z), umfassend die folgenden Schritte:
a) Bereitstellen eines Formkörpers für dentale Restaurationen, wobei der Formkörper einen Grundfarbwert FW(F) aufweist und wobei der Grundfarbwert FW(F) des Formkörpers bezüglich des Zielfarbwerts FW(Z) der dentalen Restauration einen maximalen Differenzwert ΔE von 5 aufweist;
b) Aufbringen einer Glasur auf den Formkörper, wobei die Glasur einen Farbwert FW(G) aufweist;
c) Wärmebehandeln des glasierten Formkörpers unter Erhalt der dentalen Restauration,
**dadurch gekennzeichnet, dass** der Farbwert FW(G) der Glasur der Differenz ΔE zwischen den Farbwerten FW(Z) und FW(F) entspricht.

2. Verfahren gemäß Anspruch 1, **dadurch gekennzeichnet, dass** es sich bei den Farbwerten um CIEL^{∗}a^{∗}b^{∗}-Werte handelt.

3. Verfahren gemäß wenigstens einem der Ansprüche 1 oder 2, **dadurch gekennzeichnet, dass** der Formkörper durch Auswahl aus einem Set von Formkörpern mit definierten Grundfarbwerten FW(F) durch Vergleich mit den zur Restauration benachbarten Zähnen im Mund des Patienten mit Hilfe eines Farbrings bereitgestellt wird.

4. Verfahren gemäß wenigstens einem der vorangehenden Ansprüche, **dadurch gekennzeichnet, dass** es sich bei den Grundfarbwerten um solche handelt, die den Farbwerten in den standardisierten Farbringen zur Zahnfarbenbestimmung entsprechen.

5. Verfahren gemäß wenigstens einem der vorangehenden Ansprüche, **dadurch gekennzeichnet, dass** die Bestimmung von ΔE und/oder die Auswahl des Formkörpers computergestützt erfolgt.

6. Verfahren gemäß wenigstens einem der vorangehenden Ansprüche, **dadurch gekennzeichnet, dass** die Glasur in einer Dicke von 10 bis 50 µm, vorzugsweise 20 bis 40 µm auf den Formkörper aufgetragen wird.

7. Glasur zur Verwendung in einem Verfahren gemäß wenigstens einem der Ansprüche 1 bis 6, **dadurch gekennzeichnet, dass** die Glasur
i) eine flüssige Anmischphase; und
ii) eine feste Phase umfassend färbende Substanzen
aufweist,
wobei der Anteil an flüssiger Anmischphase in der Glasur mindestens 60 Gew.-% beträgt, bezogen auf das Gesamtgewicht der Glasur, und wobei die Glasur einen definierten Farbwert FW(G) aufweist.

8. Glasur gemäß Anspruch 7, **dadurch gekennzeichnet, dass** die flüssige Anmischphase ausgewählt ist aus der Gruppe bestehend aus Wasser, einem oder mehreren Alkoholen und Mischungen hiervon, wobei der Alkohol vorzugsweise ausgewählt ist aus der Gruppe der 1-, 2- und 3-wertigen Alkohole sowie Mischungen hiervon, insbesondere aus der Gruppe bestehend aus Glycerin, 1,3-Butandiol, 1,2-Propandiol, Ethanol und Mischungen hiervon.

9. Glasur gemäß wenigstens einem der Ansprüche 7 und 8, **dadurch gekennzeichnet, dass** die flüssige Anmischphase weiterhin einen oder mehrere Komplexbildner, vorzugsweise ausgewählt aus der Gruppe bestehend aus Ethylendiamintetraessigsäure und Citronensäure, aufweist.

10. Glasur gemäß wenigstens einem Ansprüche 7 bis 9, **dadurch gekennzeichnet, dass** die Glasur in Form eines Kits vorliegt, das Kit umfassend einen ersten Behälter umfassend die flüssige Anmischphase und einen zweiten Behälter umfassend die feste Phase mit färbenden Substanzen.

11. Kit zur Herstellung einer dentalen Restauration umfassend
a) ein Sortiment an Glasuren für Formkörper für dentale Restaurationen, wobei jede Glasur einen definierten Farbwert F(Gₓ) aufweist, wobei die Farbwerte F(Gₓ) den Differenzen ΔE zwischen den Farbmustern eines standardisierten Farbrings zur Zahnfarbenbestimmung entsprechen und wobei ΔE nicht größer als 5 ist; und
b) einen Farbführer zur Zuordnung eines ermittelten ΔE-Werts zu der entsprechenden Glasur.

12. Kit gemäß Anspruch 11, **dadurch gekennzeichnet, dass** das Sortiment 2 oder mehr, vorzugsweise 3 oder mehr, besonders bevorzugt 4 oder mehr Glasuren und/oder nicht mehr als 10, vorzugsweise nicht mehr als 8, besonders bevorzugt nicht mehr als 6 Glasuren umfasst.

13. Kit gemäß wenigstens einem der Ansprüche 11 oder 12, **dadurch gekennzeichnet, dass** das Kit weiterhin einen Applikator zum Aufbringen der Glasur auf einen Formkörper für dentale Restaurationen umfasst.

14. Kit gemäß wenigstens einem der Ansprüche 11 bis 13, **dadurch gekennzeichnet, dass** es sich bei den Glasuren um Glasuren gemäß wenigstens einem der Ansprüche 5 bis 10 handelt.

15. Verwendung einer Glasur mit einem definierten Farbwert FW(G) in der Herstellung dentaler Restaurationen, wobei der Farbwert FW(G) der Differenz ΔE zwischen den Farbmustern eines standardisierten Farbrings zur Zahnfarbenbestimmung entspricht und wobei ΔE nicht größer als 5 ist.
